# EUROPEAN PATENT SPECIFICATION

(11) **EP 1 236 724 B1**
(45) Date of publication and mention of the grant of the patent: **17.08.2005**
(21) Application number: 00979015.5
(22) Date of filing: 29.11.2000
(51) Int. Cl.: C07D 311/62, A61K 31/352, A61P 39/02, A23L 1/30

(54) **NEUTRALIZING AGENT FOR TOXIN OF MICROORGANISM BELONGING TO THE GENUS CLOSTRIDIUM**
NEUTRALISIERUNGSMITTEL FÜR TOXIN DER ZUR GATTUNG CLOSTRIDIUM GEHÖRENDE MIKROORGANISMEN
AGENT DE NEUTRALISATION POUR TOXINE DE MICRO-ORGANISME APPARTENANT AU GENRE CLOSTRIDIUM

(30) Priority: 29.11.1999 JP 33882999
(43) Date of publication of application: 04.09.2002
(73) Proprietor: Japan Science and Technology Agency, Kawaguchi-shi, Saitama 332-0012 (JP)
(72) Inventor: NISHIMURA, Masakazu, Obihiro-shi, Hokkaido 080-0834 (JP); SHIMIZU, Yoshio, Obihiro-shi, Hokkaido 080-0026 (JP)
(74) Representative: Calamita, Roberto
(86) International application number: PCT/JP2000/008426
(87) International publication number: WO 2001/040213

(56) References cited:
- EP-A1- 0 397 914
- DATABASE WPI Section Ch, Week 199101 Derwent Publications Ltd., London, GB; Class B05, AN 1991-002162 XP002230091 & JP 02 276562 A (MITSUI NORIN KK), 13 November 1990 (1990-11-13)
- SANTOSH K. KATIYAR AND HASAN MUKHTAR: 'Inhibition of phorbol ester tumor promoter 12-O-tetradecynoylphorbol-13-acetate-caused inflammatory responses in SENCAR mouse skin by black tea polyphenols' CARCINOGENESIS vol. 18, no. 10, 1997, pages 1911 - 1916, XP002936745
- MASAKAZU NISHIMURA ET AL.: 'Botulinum shinkei dokuso ni yoru chudoku bougyohou no kaihatsu kenkyu' HOKKAIDO NO KENKYU KAIHATSU vol. 1999, 1999, pages 107 - 109, XP002936746

## Description

### Technical Field

The invention of the present application relates to a neutralizing agent for the toxin of *Clostridium* microorganisms. More specifically, the invention of the present application relates to a neutralizing agent for the toxin of *Clostridium* microorganisms, and pharmaceuticals or foods and drinks containing same.

### Background Art

Tea, which is regularly consumed all over the world, has been known to have various effects from old-times. In line with the recent health-boom, the capabilities of tea has attracted a great deal of attention and active studies are currently underway in such fields as chemistry and medicine.

Although there are various types of tea, including green tea, oolong tea, and black tea, tea leaves are generally obtained from *Camellia sinensis* (L) O. Kuntze, a perpetual evergreen tree belonging to the genus *theaceous camellia,* and are classified into various names according to their processing methods.

Tea leaves contain various ingredients, including organic substances such as proteins, free amino acids, caffeine, alkaloids, polyphenols, carbohydrates, organic acids, fat, pigments such as chlorophyll and carotinoids, and vitamins, as well as soluble and insoluble inorganic substances. These ingredients vary greatly depending on the processing methods of the tea leaves, and ingredients that are extracted by hot water or contained in the aroma are even more diverse.

Tea contains catechin, which is a type of polyphenol, and enzymes such as polyphenol oxidase that oxidize the catechins. Green tea is obtained when the enzymes are deactivated by heating in the early stage of the processing of tea leaves, while black tea is obtained by allowing the enzymes to fully oxidize the catechins.

Green tea is referred to as non-fermented tea, and is obtained by first heating the tea leaves by steam or panning to deactivate the enzymes such as polyphenol oxidase, and then drying them. The main components of green tea are amino acids such as theanine, glutamic acid, and aspartic acid; catechins such as epigallocatechin gallate, epicatechin gallate, epigallocatechin, and epicatechin; caffeine; sugars such as sucrose, glucose, and fructose; and chlorophyll, which is the source of the green color of tea leaves, and the color, flavor, and aroma of green tea varies depending on the balance of these components. The components comprising the aroma of green tea include various compounds such as dimethylsulfide, phenylethylalcohol, benzylalcohol, β-ionone, nerolidol, 4-vinylphenol, pyrazines, and pyrrols, and constitute its fragrance.

On the other hand, black tea is referred to as fermented tea, and is obtained by spreading out the tea leaves and evaporating water at a low temperature to activate the enzymes and soften the leaves, followed by addition of pressure to expose components such as catechins to air for the promotion of oxidation. The unique red-brown color of black tea is due to pigments such as theaflavin, which is formed by the oxidation of catechins such as epicatechin and epigallocatechin by polyphenol oxidase; thearubigin, which is a polymer formed by the further oxidation of theaflavin; and high molecular-weight oxidation polymers formed by even further oxidation polymerization of thearubigin.

Although raw leaves contain only a small amount of alcohol, processing significantly increases the amount of linallol, geraniol, methyl salicylate, ionone compounds, lactones, trance-2-hexenal, a heat-aroma component formed from sugars and amino acids, and the like.

In addition to these, there are various other types of tea, such as roasted green tea and red tea, which are obtained by the secondary processing of green tea; oolong tea, which is known as half-fermented tea; and those blended with various plants. Each contain various components, as well as a variety of components that are extracted with hot water, and different aromatic components; the effect of each component have received attention and are being actively investigated.

In particular, it has recently been scientifically proven that polyphenols such as catechin, tannin, and tannic acid have various effects such as antioxidant effect, antibacterial effect, inhibitory effects against the elevation of cholesterol, lipids, blood pressure, and blood sugar, and antitumor effect, and various products containing catechins and tannin, such as health foods, drinks, medicines, deodorants, and antibacterial agents, are being searched and developed.

However, except for catechin and tannin, very little is known about the large number of components found in tea. Especially regarding the antibacterial effects, it is unclear as to what kindof component shows effect against whichparticular microorganism or toxin. Therefore, its understanding has been limited to the fact that green tea shows the highest antibacterial effect, and specific components that show neutralizing effect against specific bacteria is yet to be known.

Among the various microorganisms that are hazardous to the human body, *Clostridium* botulinum and *Clostridium* tetani are both anaerobic bacteria abundant in soil that belong to the genus *Clostridium*, and the toxins produced by them are known to be similar with almost identical molecular weights. The toxins of these *Clostridium* microorganisms cause paralyzing effects on the motoneuronal skeletal muscle system with a very high death rate, and preventing infection is a necessity.

*Clostridium* botulinum forms spores particularly resistant to heat and disinfectants, grows in, for instance, food, at 3-40°C, pH 4.5, without oxygen, in the presence of water and nutrients, and produces toxin. The latent period of the bacteria is 12-36 hours, but the toxin produced is a potential neurotoxin that, if ingested, causes exhaustion, dizziness, and gastrointestinal symptoms such as nausea and vomiting, which then gradually leads to nervous disorders such as headache, visual impairment, difficulty in swallowing, and difficulty in walking, and if serious, eventually leads to dyspnea and results in death.

Although many cases of botulism in Japan are caused by fish "*izushi,*" cases caused by imported bottled or canned foods, ham, sausage, and the like have often occurred in recent years, and botulism antitoxin is usually administered as a remedy.

On the other hand, *Clostridium* tetani is a gram-positive anaerobe that exists not only in soil and water but also in human intestinal tracts. However, when entered from a contaminated external wound, such as a traffic accident injury or punctures caused by bamboo, pieces of wood, or old nails, a toxin is produced and released into the blood stream. The latent period of *Clostridium* tetani is normally 1-2 weeks, and when the toxin is produced, it initially causes symptoms of neural convulsion such as trismus and canine laugh, gradually leading to convulsion and ankylosis of all body muscles, and causes dyspnea, resulting in death. The death rate of infection by this toxin is as high as 70-80%.

To prevent tetanus, vaccination is the most effective and is enforced. Although mixed vaccines are also used today, adults are generally vaccinated with tetanus toxoid. However, because additional vaccination is required, and if infected more than five years after the additional vaccination, subcutaneous injection of tetanus toxoid is also required, relatively few people are actually vaccinated for tetanus.

Therefore, infection by such *Clostridium* microorganisms are often recognized only after symptoms caused by the toxins appear, thus exposing patients to highly dangerous conditions and requiring immediate medical treatment by a physician.

Also, food poisoning is dreaded especially among food-processing businesses, and various methods of sterilization and disinfection are being studied and implemented. However, as described above, *Clostridium* botulinum loses its toxicity only after a long period of treatment at high-temperature, which may not be applicable for some types of food. Further, *Clostridium* botulinum prefers anaerobic conditions found in preserved foods such as bottled and canned preserves, making its prevention even more difficult.

On the other hand, there is another problem in that although various neutralizing agents and disinfectants are being developed, most neutralizing agents and disinfectants that are highly effective against these toxins also tend to be harmful to the human body. In recent years, especially due to problems such as allergies, repulsion against food additives are spreading among the public, and foods with few or no additives are favored.

Therefore, an easy and effective method of safely neutralizing or removing the *Clostridium* microorganisms and the deadly neurotoxins they produce have not been known.

Hence, the invention of the present application has been developed in view of the facts described above, and the object of the present invention is to solve the problems of the conventional technologies and to provide a safe and convenient neutralizing agent that is effective against the toxins produced by *Clostridium* microorganisms using tea leaf-extracts as its effective component.

### Disclosure of the Invention

In order to solve the problems of the conventional technologies, the following inventions are provided.

In other words, the invention of the present application firstly provides a neutralizing agent for toxins produced by *Clostridium* microorganisms, comprising thearubigin as its effective component.

Also, for the above-described first invention, the invention of the present application secondly provides the neutralizing agent for toxins produced by *Clostridium* microorganisms, wherein the thearubigin is a type of brown-colored pigment contained in tea leaves.

For the first and second inventions described above, the invention of the present application provides, thirdly, the neutralizing agent for toxins produced by *Clostridium* microorganisms, wherein the thearubigin is a water or hot-water extract of tea leaves, and fourthly, the neutralizing agent for toxins produced by *Clostridium* microorganisms, wherein thearubigin is an alcohol extract of tea leaves obtained after extraction by water or hot water.

Also, the invention of the present application fifthly provides the neutralizing agent for toxins produced by *Clostridium* microorganisms, wherein the thearubigin in the first to forth invention is a type of oxidative polymer of theaflavin represented by the following chemical formula [1] 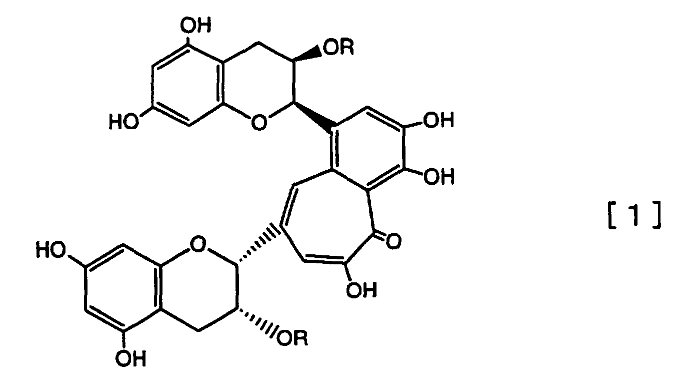 (wherein R is a hydrogen atom or galloyl group).

Further, the invention of the present application sixthly provides the neutralizing agent for toxins produced by *Clostridium* microorganisms, wherein the thearubigin in the fifth invention is a type of oxidative polymer of theaflavin represented by the following chemical formula [2] 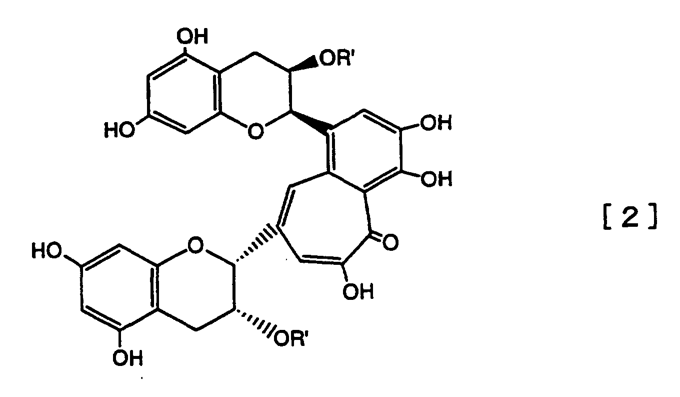 (wherein R' is a hydrogen atom or galloyl group) with a structure of the following formula [3] 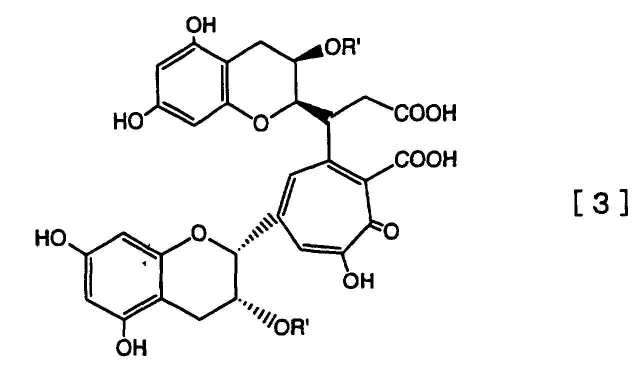 (wherein R is a hydrogen atom or galloyl group).

Still further, the invention of the present application seventhly provides a pharmaceutical comprising any of the above neutralizing agent for toxins produced by *Clostridium* microorganisms, and eighthly, a food or drink comprising any one of the above the neutralizing agent for toxins produced by *Clostridium* microorganisms as an additive.

### Brief Description of Drawings

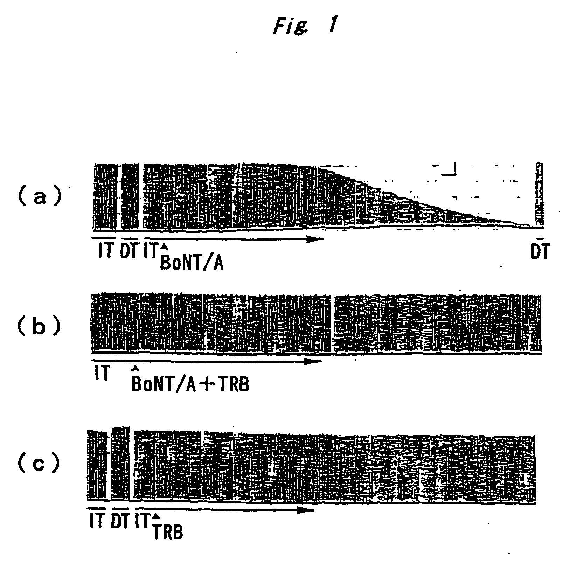

Fig.1 is a diagram showing the reaction of a motoneuronal skeletal muscle system infected by *Clostridium* botulinum to electric stimulation. Here, IT represents muscle contraction against electric stimulation of the nerve trunk, DT represents muscle contraction on direct electric stimulation of the skeletal muscle system, a represents the system to which botulinum neurotoxin (15 µl of a solution BoNT/A 1 mg/ml was added to a 20 ml test tube to make a solution of 1.5 nM) was added, b represents the system to which a mixture of botulinum neurotoxin and thearubigin (15 µl of a solution of BoNT/A 1 mg/ml) was added, and c represents the system to which only thearubigin (10 µl of the extract solution) was added.

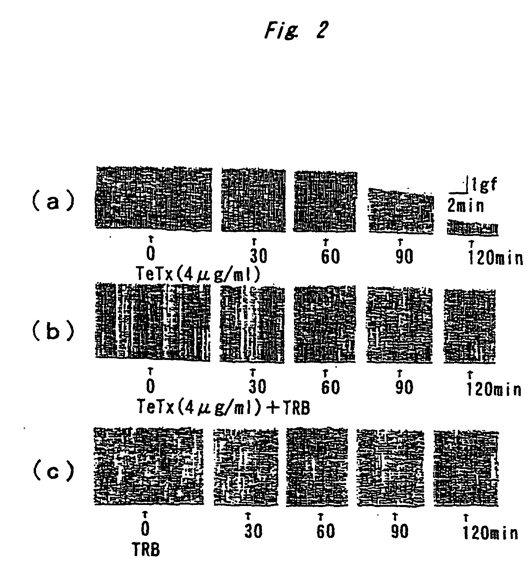

Fig.2 is a diagram showing the reaction of nerve trunk of the motoneuronal skeletal muscle system to electric stimulation in a test tube to which tetanus toxin (330 µl of a solution of TeTx 1 mg/ml was added to a 20 ml test tube to make a solution of 4 µg/ml) was added. Here, a represents the system to which tetanus toxin was added, b represents the system to which a mixture of tetanus toxin and thearubigin (330 µl of a solution of TeTx 1 mg/ml) and thearubigin (10 µl of the extract solution) were added, and c represents the system to which only thearubigin (10 µl of the extract solution) was added.

### Best Mode for Carrying Out the Invention

The present inventors noted that black tea weakens the neurotoxin of *Clostridium* botulinum, and found through extensive research that thearubigin, the brown-colored pigment contained in tea leaves, has a function of neutralizing the toxin produced by *Clostridium* botulinum, and through further examination of these findings, developed the present invention.

As described earlier, the number of ingredients contained in tea leaves that are known today is enormous, but the neutralizing agent for the toxins of *Clostridium* microorganisms of the invention of the present application comprises thearubigin as its effective ingredient. Here, the effective ingredient, thearubigin, has been reported as one type of brown-colored pigment contained in black tea and the like. It has also been reported as an oxidative polymer of theaflavin, the compound [1] described earlier.

Theaflavin and thearubigin are said to be formed through the oxidation of catechins in tea leaves by polyphenol oxydase, and thearubigin is considered to be a polymer resulting from such oxidation. Although the structure of thearubigin has not been completely clarified, a compound having the structure of the following chemical formula [3] 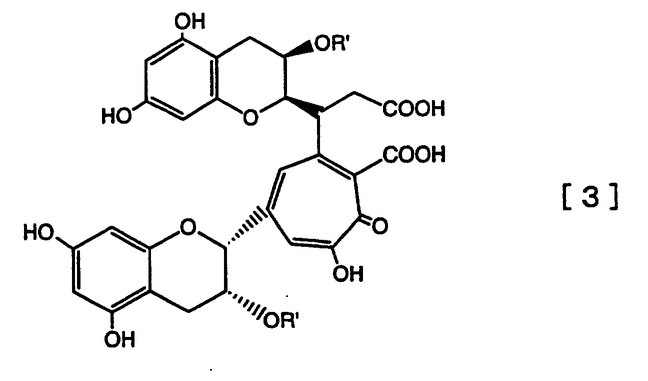 (wherein R is a galloyl group) has been proposed by Katiyar et al. (Katiyar, S.K. and Mukhtar, H., Caroinogenesis 18, 1911-16 (1997)). Here, the galloyl group is a substituent represented by the following chemical formula [4].

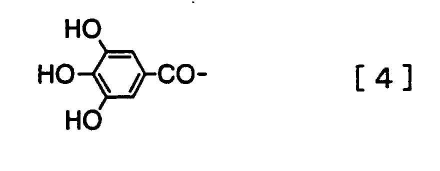

The neutralizing agent for the toxins of *Clostridium* microorganisms of the present invention contains the above described thearubigin as an effective ingredient, and is more specifically exemplified as the water or hot-water extract of tea leaves, especially tea leaves containing the brown-colored pigments such as black tea. In this case, the extract is preferably that of fermented tea obtained by increasing the enzymatic activity and oxidizing catechins during the processing of tea leaves. Further, black tea is most preferable as such fermented tea. In addition, the neutralizing agent for the toxins of *Clostridium* microorganisms of the present invention may be the extract from tea leaves such as roasted green tea obtained by the secondary processing of non-fermented tea.

Extraction with water or hot water is carried out, for example, using water of an ambient temperature (approximately 10-30°C) or hot water (above 30°C to boiling) . More effectively, extraction is carried out using hot water of 60ºC or above.

If necessary, the extracted aqueous phase may further be extracted using alcohol following washing with organic solvents such as esters.

More specifically, in the invention of the present application, as described in the following examples, after estraction by water or hot water, contents extracted by alcohol, preferably alcohols such as butanol, isopropanol, isobutanol, hexanol, in particular butanol, are exemplified as suitable neutralizing agents.

Although the neutralizing agent for the toxins of *Clostridium* microorganisms of the present invention is effective for any *Clostridium* microorganism, it is especially effective in neutralizing the neurotoxins produced by *Clostridium* botulinum and *Clostridium* tetani. Incidentally, the term "neutralization" used herein refers to the inhibition, reduction, and annihilation of any negative function of the toxins produced by microorganisms belonging to the genus *Clostridium*.

The neutralizing agent for the toxins of *Clostridium* microorganisms of the present invention can be used, for example, to detoxify the *Clostridium* microorganism toxins in foods contaminated with *Clostridium* mocroorganisms, as medicine such as a cleaning agent or disinfectant, which may be applied on wounds infected by *Clostridium* microorganisms and its toxins by spreading, spraying or wiping.

Further, the neutralizing agent for the toxins of *Clostridium* microorganisms of the present invention may be added during the production process of various foods, and be used as a preventive agent for food poisoning caused by *Clostridium* microorganism toxins. Examples of such foods are canned foods, bottled foods, ham, sausage, *kamaboko* (fish sausage), *izushi*, and honey, but are not limited to these. Further, the addition of this neutralizing agent may be done at any step of food production including processing and wrapping.

Since the thearubigin contained in the neutralizing agent for the *Clostridium* microorganism toxins of the present invention is a natural pigment extracted from tea leaves, the neutralizing agent of the present invention is harmless to humans and the environment; therefore, it is highly safe and may preferably be used as a pharmaceutical or food additive.

The embodiments of the present invention is described in more detail by the following Examples, with reference to the attached drawings. It is needless to say, that the invention of the present application is not limited by the following examples, and that various details are available.

### Examples

### <Preparation 1> Separative extraction of thearubigin

To 12 g of black tea leaves, 90 ml of hot water (boiling water) was added and left for 2 minutes, after which it was filtrated through a Whatman No. 2 filter. To 40 ml of the extract obtained, 40 ml of chloroform was added, and after 3 minutes of vigorous stirring, the aqueous layer was separated from the organic layer. To 40 ml of the aqueous layer, 40 ml of ethyl acetate was added, and vigorously stirred for 3 minutes. Once again, the aqueous and organic layers were separated, and 1-butanol was added to 40 ml of the aqueous layer and vigorously stirred for an additional 3 minutes. The organic layer (1-butanol layer) alone was recovered and evaporated to dryness in a rotary evaporator to obtain the extract (thearubigin = TRB). The thearubigin obtained was dissolved in 3 ml of water to prepare a TRB solution.

### <Preparation 2> Preparation of live specimens of the motoneuronal skeletal muscle system and confirmation of skeletal muscle contraction

Live specimens of the motoneuronal skeletal muscle system were prepared, and contraction of the skeletal muscle in reaction to electrostimulation applied to the nerve trunk in a test tube, as well as similar contraction of the skeletal muscle in reaction to a direct electrostimulation applied to the skeletal muscle, were confirmed.

### <Example 1> Neutralizing effect of thearubigin against botulinum neurotoxin (1)

Botulinum neurotoxin (BoNT), a mixture of BoNT and TRB, and TRB alone were separately added to test tubes containing the prepared live specimens of the motoneuronal skeletal muscle system.

Fig. 1 shows the reaction of the motoneuronal skeletal muscle system to electrostimulation. (IT: muscle contraction by electrostimulation of the nerve trunk; DT: muscle contraction when electrostimulation was directly applied to the skeletal muscle.)

Muscle contraction reaction to direct electrostimulation of the skeletal muscle was not inhibited by BoNT, but muscle contraction reaction to electrostimulation of the nerve trunk was found to be inhibited (Fig. 1a).

On the other hand, the muscle contraction reaction to electrostimulation of the nerve trunk was not inhibited by the mixture of BoNT and TRB (Fig. 1b).

Further, TRB alone did not affect the contraction of the skeletal mustle (Fig.1c).

Therefore it was confirmed that thearubigin neutralizes botulinum neurotoxin. Also, thearubigin alone was found to show no effect on the contraction of the motoneuronal skeletal muscle systems.

### <Example 2> Neutralization effect of thearubigin against tetanus toxin

In a similar manner as in Example 1, tetanus toxin (TeTx), TeTX and TRB, and TRB alone were added respectively to live specimens of the motoneuronal skeletal muscle system, and reactions of the motoneuronal skeletal muscle system against electrostimulation were compared.

Fig. 2 shows the reaction of the motoneuronal skeletal muscle system to electrostimulation. Muscle contraction due to electrostimulation of the nerve trunk was inhibited when TeTx was added (Fig.2a), while muscle contraction was found to occur as usual when TeTX and TRB were added (Fig. 2b). Further, muscle contraction against electrostimulation was not inhibited by TRB alone either (Fig. 2c).

Therefore, it was confirmed that thearubigin exhibits a neutralization effect against tetanus toxin, as well.

### <Examples 3 and 4> and <Comparative Examples 1-4> Botulinum neurotoxin neutralization effect of tea-leaf extracts

In a similar manner as in Examples 1 and 2, the muscle contraction reaction of the motoneuronal skeletal muscle system to electrostimulation was compared for samples with hot-water extracts of black tea (Example 3) and roasted green tea (Example 4) added with BoNT, and a sample with BoNT alone.

Further, the muscle contraction reaction of the motoneuronal skeletal muscle system to electrostimulation was studied in a similar manner when green tea (Comparative Example 1), tannic acid (Comparative Example 2), catechin (Comparative Example 3), and theaflavin (Comparative Example 4) were added with BoNT, respectively.

Table 1 shows the botulinum neurotoxin neutralization effect of each component.

**Table 1:**

| BoNT neutralization effects of tea-leaf extract ingredients | | | |
|---|---|---|---|
| | | Extracted Component | Effect |
| Example | 1, 2 | Thearubigin | + |
| | 3 | Black Tea | + |
| | 4 | Roasted Green Tea | + |
| Comparative Example | 1 | Green Tea | - |
| | 2 | Tannic Acid | - |
| | 3 | Catechin | - |
| | 4 | Theaflavin | - |
| +: Effective in neutralization | | | |
| ―: Ineffective in neutralization | | | |

The systems to which black tea, roasted green tea, or thearubigin was added showed BoNT neutralizing effect, but green tea, tannic acid, catechin, and the aflavin showed no neutralizing effect. In particular, the BoNT neutralizing effect was high for the system to which black tea was added. This result indicates that black-tea extracts exhibit a high neutralizing effect for botulinum neurotoxin, and since extracts of black tea and roasted green tea that exhibit neutralizing effect contain, as at least one type of brown-colored pigment, thearubigins, it was suggested that these exhibit neutralizing effect for botulinum neurotoxin.

### <Example 5> Neutralizing effect of thearubigin for botulinum neurotoxin (2)

A dose of 100 µl (= 100 µg) of 1 mg/ml botulinum neurotoxin dissolved in 0.05 M acetate-0.2 M sodium chloride buffer solution (pH 6.0) were orally administered to each mouse. Mixed TRB (the solution extracted above) was orally administered alone at equal volume (1 times volume extract), twice the volume extract, or 4 times the volume extract, or as a mixture with BoNT (mixing ratio: 1, 2, and 4). The results are shown in Table 2.

**Table 2:**

| Survival rate of mice with oral administration of BoNT and TRB | |
|---|---|
| Addition | number of survivor/number of test |
| TRB (1) | 8/8 |
| TRB (2) | 8/8 |
| TRB (4) | 12/12 |
| BoNT/A(1) | 0/8 |
| BoNTIB (1) | 0/8 |
| BoNT/E (1) | 0/8 |
| BoNT/A (1) + TRB (1) | 0/8 |
| BoNT/A (1) + TRB (2) | 3/8 |
| BoNT/A (1) + TRB (4) | 8/8 |
| BoNT/B (1) + TRB (4) | 8/8 |
| BoNT/E (1) + TRB (4) | 8/8 |
| BoNT : 100 µg/100 µl/individual | |
| TRB (n) : n × volume of Thearubigin | |
| BoNT : Botulinum neurotoxin | |
| /A,/B,/E : A, B, E type | |

All mice to which BoNT (A, B, and E types) was orally administered were killed. On the other hand, 100% of the mice to which mixtures of BoNT (A, B, and E types) and TRB (4 times volume BoNT) were orally administered survived.

Since all mice to which BoNT (A type) and an equal volume of TRB were administered were killed, and 3/8 of the mice to which BoNT and twice as much TRB were administered survived, TRB was found to exhibit a BoNT neutralizing effect without fail when administered at approximately 4 times the volume of the toxin. On the other hand, all mice to which TRB alone was orally administered survived, indicating that TRB shows no toxicity at this dose range.

### Industrial Applicability

As described above in detail, the present invention provides a neutralizing agent for toxins of *Clostridium* microorganisms, which can neutralize toxins of *Clostridium* microorganisms such as *Clostridium* botulinum and *Clostridium* tetani, is highly safe against living organisms and the environment, and which can be used easily.

## Claims

1. A neutralizing agent for toxins produced by *Clostridium* microorganisms, comprising thearubigin as its effective component.

2. The neutralizing agent for toxins produced by *Clostridium* microorganisms of claim 1, wherein the thearubigin is at least one type of brown-colored pigment component of tea leaves.

3. The neutralizing agent for toxins produced by *Clostridium* microorganisms of claims 1 or 2, wherein the thearubigin is a water or hot-water extract of tea leaves.

4. The neutralizing agent for toxins produced by *Clostridium* microorganisms of claims 1 or 2, wherein thearubigin is an alcohol extract of tea leaves obtained after extraction by water or hot water.

5. The neutralizing agent for toxins produced by *Clostridium* microorganisms of any one of claims 1 to 4, wherein the thearubigin is a type of oxidative polymer of theaflavin represented by the following chemical formula [1] 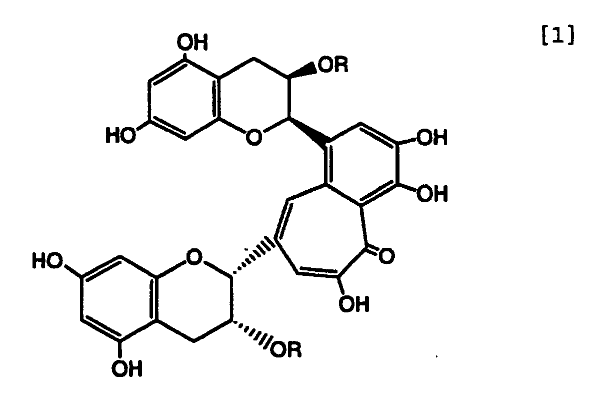 (wherein R is a hydrogen atom or galloyl group).

6. The neutralizing agent for toxins produced by *Clostridium* microorganisms of any one of claims 1 to 5, wherein the thearubigin is a type of oxidative polymer of theaflavin represented by the following chemical formula [2] 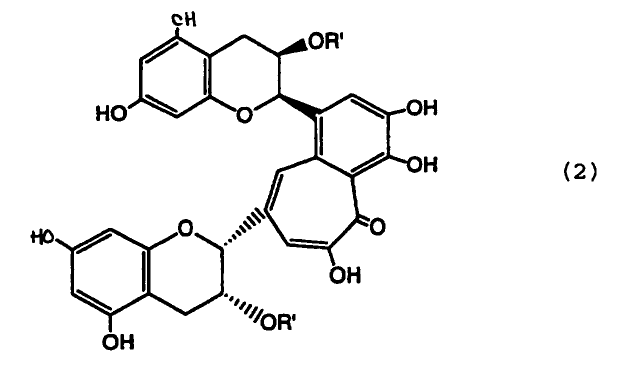 (wherein R' is a hydrogen atom or galloyl group) with a structure of the following formula [3] 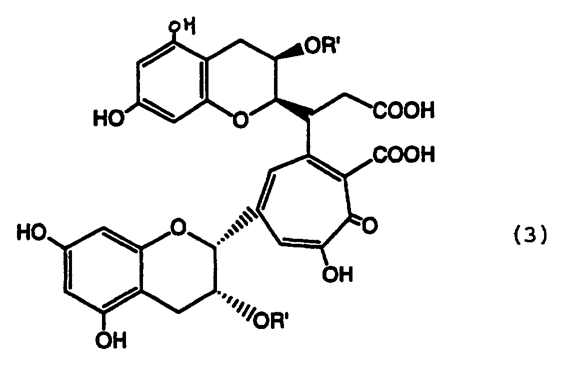 (wherein R is a hydrogen atom or galloyl group).

7. A neutralizing agent for toxins produced by *Clostridium* microorganisms obtainable by a process comprising the steps of:
extracting fermented tea leaves with water; and
extracting the water extract with an alcohol,
wherein said agent comprises thearubigin.

8. A food or drink comprising, as an additive, the neutralizing agent for toxins produced by *Clostridium* microorganisms of any one of claims 1 to 7.

9. Use of the neutralizing agent for toxins produced by *Clostridium* microorganisms of any one of claims 1 to 7 as a food additive.

10. A pharmaceutical composition comprising the neutralizing agent for toxins produced by *Clostridium* microorganisms of any one of claims 1 to 6.

11. A pharmaceutical composition as claimed in claim 10 wherein said composition is a topical pharmaceutical composition for application to wounds by spreading, spraying or wiping.

12. A neutralizing agent for toxins produced by *Clostridium* microorganisms as claimed in any one of claims 1 to 7 or a pharmaceutical composition as claimed in claim 10 or claim 11 for use as a cleaning agent or disinfectant for application to wounds infected by *Clostridium* microorganisms and its toxins by spreading, spraying or wiping.

13. Use of the neutralizing agent for toxins produced by *Clostridium* microorganisms of any one of claims 1 to 7 for the manufacture of a medicament for inhibiting, reducing and annihilating any of the negative effects of the toxins produced by *Clostridium* microorganisms.

## Patentansprüche

1. Neutralisierendes Mittel für von *Clostridium*-Mikroorganismen herrührende Toxine, das Thearubigin als seine wirksame Komponente beinhaltet.

2. Neutralisierendes Mittel für von *Clostridium*-Mikroorganismen herrührende Toxine nach Anspruch 1, wobei das Thearubigin wenigstens eine Art der braunfarbigen Pigmentkomponenten von Teeblättern ist.

3. Neutralisierendes Mittel für von *Clostridium*-Mikroorganismen produzierte Toxine nach Anspruch 1 oder 2, wobei das Thearubigin ein Wasser- oder Heißwasserextrakt aus Teeblättern ist.

4. Neutralisierendes Mittel für von *Clostridium*-Mikroorganismen herrührende Toxine nach Anspruch 1 oder 2, wobei Thearubigin ein nach Extraktion mit Wasser oder Heißwasser erhaltener Alkoholextrakt aus Teeblättern ist.

5. Neutralisierendes Mittel für von *Clostridium*-Mikroorganismen herrührende Toxine nach einem der Ansprüche 1 bis 4, wobei das Thearubigin eine Art der oxidativen Polymere von Theaflavin der allgemeinen chemischen Formel [1] 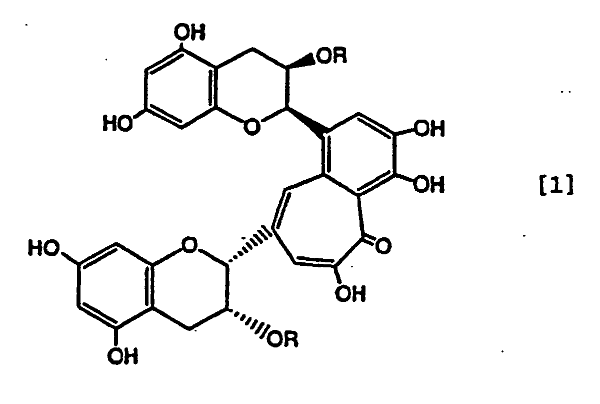 (worin R für ein Wasserstoffatom oder eine Galloyl-Gruppe steht) ist.

6. Neutralisierendes Mittel für von *Clostridium*-Mikroorganismen herrührende Toxine nach einem der Ansprüche 1 bis 5, wobei das Thearubigin eine Art oxidatives Polymer von Theaflavin der allgemeinen chemischen Formel [2] 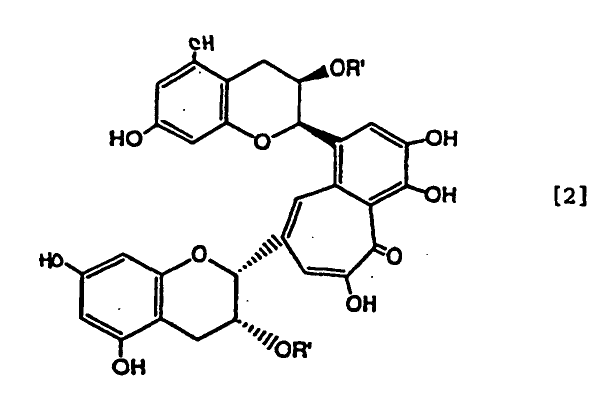 (worin R' für ein Wasserstoffatom oder eine Galloyl-Gruppe steht)
mit einer Struktur der folgenden Formel [3] 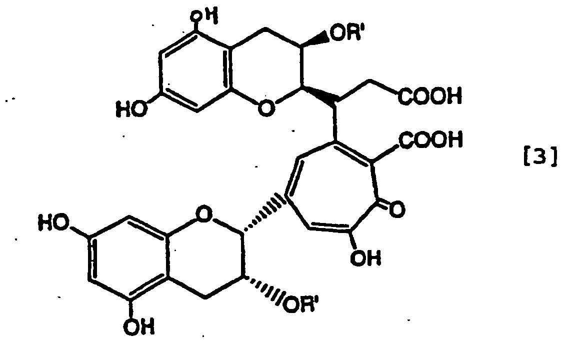 (worin R für ein Wasserstoffatom oder eine Galloyl-Gruppe steht) ist.

7. Neutralisierendes Mittel für von *Clostridium*-Mikroorganismen herrührende Toxine, erhältlich mit einem Verfahren, wobei man:
fermentierte Teeblätter mit Wasser extrahiert; und
den Wasserextrakt mit einem Alkohol extrahiert,
wobei das Mittel Thearubigin beinhaltet.

8. Nahrungsmittel oder Getränk, welches das neutralisierende Mittel für von *Clostridium*-Mikroorganismen herrührende Toxine nach einem der Ansprüche 1 bis 7 als Zusatz beinhaltet.

9. Verwendung des neutralisierenden Mittels für von *Clostridium*-Mikroorganismen herrührende Toxine nach einem der Ansprüche 1 bis 7 als Zusatz für Nahrungsmittel.

10. Pharmazeutische Zusammensetzung, die das neutralisierende Mittel für von *Clostridium*-Mikroorganismen herrührende Toxine nach einem der Ansprüche 1 bis 6 beinhaltet.

11. Pharmazeutische Zusammensetzung nach Anspruch 10, wobei die Zusammensetzung eine topische pharmazeutische Zusammensetzung zur Anwendung an Wunden durch Streichen, Sprühen oder Wischen ist.

12. Neutralisierendes Mittel für von *Clostridium*-Mikroorganismen herrührende Toxine nach einem der Ansprüche 1 bis 7 oder pharmazeutische Zusammensetzung nach Anspruch 10 oder 11 zur Verwendung als Reinigungsmittel oder Desinfiziermittel zur Anwendung an Wunden, die mit *Clostridium*-Mikroorganismen und ihren Toxinen infiziert sind, durch Streichen, Sprühen oder Wischen.

13. Verwendung des neutralisierenden Mittels für von *Clostridium*-Mikroorganismen herrührende Toxine nach einem der Ansprüche 1 bis 7 zur Herstellung eines Medikaments zum Hemmen, Vermindern und Aufheben einer beliebigen negativen Wirkung der von *Clostridium*-Mikroorganismen herrührenden Toxine.

## Revendications

1. Agent de neutralisation pour toxines produites par des micro-organismes du genre *Clostridium,* comprenant de la théarubigine à titre de son composant efficace.

2. Agent de neutralisation pour toxines produites par des micro-organismes du genre *Clostridium* selon la revendication 1, dans lequel la théarubigine est au moins de type composant de pigment de coloration brune des feuilles de thé.

3. Agent de neutralisation pour toxines produites par des micro-organismes du genre *Clostridium* selon les revendications 1 ou 2, dans lequel la théarubigine est un extrait de feuilles de thé obtenu au moyen d'eau ou d'eau chaude.

4. Agent de neutralisation pour toxines produites par des micro-organismes du genre *Clostridium* selon les revendications 1 ou 2, dans lequel la théarubigine est un extrait alcoolique de feuilles de thé obtenu après extraction au moyen d'eau ou d'eau chaude.

5. Agent de neutralisation pour toxines produites par des micro-organismes du genre *Clostridium* selon l'une quelconque des revendications 1 à 4, dans lequel la théarubigine est de type polymère d'oxydation de la théaflavine représenté par la formule chimique [1] suivante 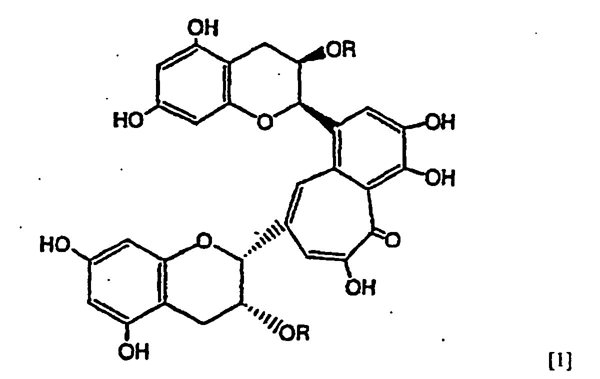 (dans laquelle R représente un atome d'hydrogène ou un groupe galloyle).

6. Agent de neutralisation pour toxines produites par des micro-organismes du genre *Clostridium* selon l'une quelconque des revendications 1 à 5, dans lequel la théarubigine est de type polymère d'oxydation de la théaflavine représenté par la formule chimique [2] suivante 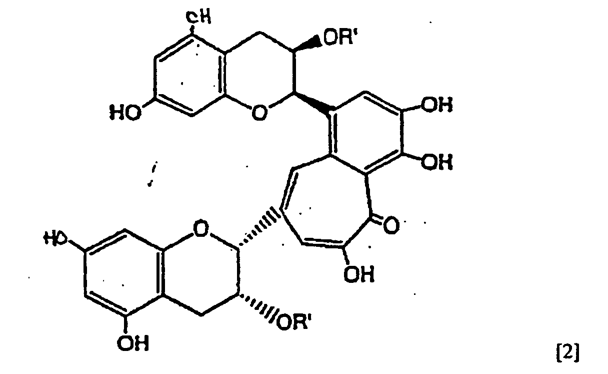 (dans laquelle R' représente un atome d'hydrogène ou un groupe galloyle) avec une structure de formule [3] suivante 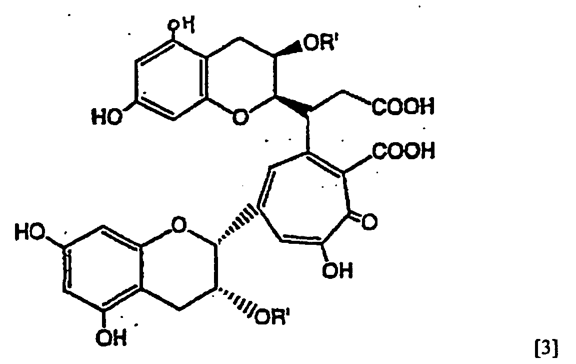 (dans laquelle R représente un atome d'hydrogène ou un groupe galloyle).

7. Agent de neutralisation pour toxines produites par des micro-organismes du genre *Clostridium* susceptible d'être obtenu selon un procédé comprenant les étapes où :
on extrait des feuilles de thé fermentées par de l'eau, et
on extrait l'extrait aqueux par un alcool,
dans lequel ledit agent comprend de la théarubigine.

8. Aliment ou boisson comprenant, à titre d'additif, l'agent de neutralisation pour toxines produites par des micro-organismes du genre *Clostridium* selon l'une quelconque des revendications 1 à 7.

9. Utilisation de l'agent de neutralisation pour toxines produites par des micro-organismes du genre *Clostridium* selon l'une quelconque des revendications 1 à 7, à titre d'additif alimentaire.

10. Composition pharmaceutique comprenant l'agent de neutralisation pour toxines produites par des micro-organismes du genre *Clostridium* selon l'une quelconque des revendications 1 à 6.

11. Composition pharmaceutique telle que revendiquée dans la revendication 10 dans laquelle ladite composition est une composition pharmaceutique pour voie topique pour application à des blessures, par badigeonnage, pulvérisation ou essuyage.

12. Agent de neutralisation pour toxines produites par des micro-organismes du genre *Clostridium* tel que revendiqué dans l'une quelconque des revendications 1 à 7 ou composition pharmaceutique telle que revendiquée dans la revendication 10 ou la revendication 11 pour utilisation comme agent de nettoyage ou désinfectant pour application à des blessures infectées par des micro-organismes du genre *Clostridium* et ses toxines, par badigeonnage, pulvérisation ou essuyage.

13. Utilisation de l'agent de neutralisation pour toxines produites par des micro-organismes du genre *Clostridium* selon l'une quelconque des revendications 1 à 7 pour la fabrication d'un médicament pour l'inhibition, la réduction ou la suppression des effets négatifs des toxines produites par des micro-organismes du genre *Clostridium*.
